(19) Europäisches Patentamt
European Patent Office
Office européen des brevets

(11) Veröffentlichungsnummer : **0 447 963 B1**

(12) # EUROPÄISCHE PATENTSCHRIFT

(45) Veröffentlichungstag der Patentschrift :
**15.12.93 Patentblatt 93/50**

(51) Int. Cl.$^5$ : **C07C 31/20,** C07C 29/149

(21) Anmeldenummer : **91103924.6**

(22) Anmeldetag : **14.03.91**

(54) **Verfahren zur Herstellung von 1,4-Butandiol.**

(30) Priorität : **21.03.90 DE 4009029**

(43) Veröffentlichungstag der Anmeldung :
**25.09.91 Patentblatt 91/39**

(45) Bekanntmachung des Hinweises auf die Patenterteilung :
**15.12.93 Patentblatt 93/50**

(84) Benannte Vertragsstaaten :
**AT BE CH DE ES FR GB IT LI NL SE**

(56) Entgegenhaltungen :
**EP-A- 0 382 050**
**DE-A- 2 321 101**
**DE-A- 2 543 673**
**DE-A- 3 726 509**
**DE-A- 3 904 083**
**US-A- 3 361 832**
**US-A- 4 032 458**

(73) Patentinhaber : **BASF Aktiengesellschaft**
**Carl-Bosch-Strasse 38**
**D-67063 Ludwigshafen (DE)**

(72) Erfinder : **Stabel, Uwe, Dr.**
**Buchenweg 2**
**W-6803 Edingen-Neckarhausen (DE)**
Erfinder : **Gosch, Hans-Jürgen, Dr.**
**Seebacher Strasse 37**
**W-6702 Bad Duerkheim (DE)**
Erfinder : **Fischer, Rolf, Dr.**
**Bergstrasse 98**
**W-6900 Heidelberg (DE)**
Erfinder : **Harder, Wolfgang, Dr.**
**Bergwaldstrasse 16**
**W-6940 Weinheim, (DE)**
Erfinder : **Hechler, Claus, Dr.**
**Uhlandstrasse 37a**
**W-6800 Mannheim 1 (DE)**

**EP 0 447 963 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Erfindung betrifft ein Verfahren zur Herstellung von 1,4-Butandiol durch die katalytische Hydrierung von Maleinsäureanhydrid in Gegenwart von Alkoholen mit Hilfe eines kobalthaltigen Katalysators.

Es ist bekannt, 1,4-Butandiol (BD) durch die katalytische Hydrierung von Maleinsäureanhydrid (MSA) in Gegenwart von Alkoholen herzustellen. So wird in DE-A 28 45 905 beschrieben, daß sich Lösungen von MSA in einwertigen, aliphatischen Alkoholen in Gegenwart von Kupferchromit-Katalysatoren bei Temperaturen von 180 bis 300°C und bei einem Druck von 250 bis 350 bar einstufig zu 1,4-Butandiol hydrieren lassen.

DE-A 37 26 509 offenbart die einstufige Hydrierung von MSA in Gegenwart von aliphatischen Alkoholen mit Hilfe von kobalthaltigen Katalysatoren, bei Temperaturen von 100 bis 350°C und einem Druck von 50 bis 350 bar. Zwar liefern beide Verfahren im Labormaßstab gute bis sehr gute Butandiol-Ausbeuten, ihre Übertragung in den technischen Maßstab führte jedoch bislang zu großen Schwierigkeiten. Ursache hierfür ist die große Menge an freiwerdender Hydrierwärme, welche nur schwierig abgeführt werden kann und zu Selektivitätsverlusten bei der Umsetzung sowie zu einer Schädigung des Katalysators führt.

US-A 4 584 419 beschreibt ein Verfahren zur Herstellung von BD durch die katalytische Hydrierung der Di-$C_1$- bis $C_3$-alkylester von $C_4$-Dicarbonsäuren mit Hilfe eines Kupferchromit-Katalysators, wobei diese Ester bei einem Druck von 25 bis 75 bar zunächst in einer ersten Hydrierstufe bei 170 bis 190°C teilhydriert und anschließend in einer zweiten Hydrierstufe bei 160 bis 175°C nachhydriert werden. Nachteilig an diesem Verfahren ist insbesondere, daß die als Einsatzstoffe dienenden Dicarbonsäurediester erst noch in einer separaten Umsetzung, beispielsweise durch Veresterung von MSA, erzeugt werden müssen. Dieses Verfahren ist daher unwirtschaftlich.

Es bestand daher die Aufgabe, ein im technischen Maßstab anwendbares und wirtschaftliches Verfahren zur Hydrierung von MSA zu BD zu entwickeln, das insbesondere auch bei einer hohen Katalysator-Belastung eine gute BD-Selektivität und eine lange Katalysator-Standzeit ermöglicht.

Dementsprechend wurde ein Verfahren zur Herstellung von 1,4-Butandiol durch die katalytische Hydrierung von Maleinsäureanhydrid in Gegenwart von Alkoholen, bei erhöhter Temperatur und bei erhöhtem Druck und mit Hilfe eines kobalthaltigen Katalysators gefunden, das dadurch gekennzeichnet ist, daß man die Reaktionsmischung in einer ersten Hydrierstufe bei Temperaturen von 100 bis 200°C und einem Druck von 30 bis 200 bar teilhydriert und anschließend den Hydrieraustrag dieser ersten Stufe ohne weitere Aufarbeitung bei höheren Temperaturen und bei einem höheren Druck als in der ersten Hydrierstufe in einer zweiten Hydrierstufe bei Temperaturen von 200 bis 300°C und einem Druck von 200 bis 350 bar nachhydriert.

Wird MSA (I) in Alkoholen gelöst, so reagiert es bereits während des Lösevorganges unter Addition eines Alkoholmoleküls praktisch quantitativ zum betreffenden Maleinsäurehalbester II (s. Gleichung (1)).

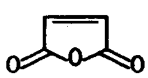

$$+ \ ROH \longrightarrow HOOC{-}HC{=}CH{-}COOR \qquad (1)$$

I      II

Bei der Hydrierung von MSA-Lösungen in Alkoholen sind somit die Halbester II das eigentlich zu hydrierende Substrat. Bei der Hydrierung wird vermutlich zunächst die Doppelbindung des Maleinsäurehalbesters hydriert (vgl. Gleichung (2)), und es entsteht der betreffende Bernsteinsäurehalbester III:

$$\underset{II}{HOOC{-}HC{=}CH{-}COOR} + H_2 \longrightarrow \underset{III}{HOOC{-}CH_2{-}CH_2{-}COOR} \qquad (2)$$

Im weiteren Verlauf der Hydrierung wird der Bernsteinsäurehalbester III unter Verbrauch von 4 Molen Wasserstoff pro Mol III zu BD IV hydriert (vgl. Gleichung (3)).

$$\underset{III}{HOOC{-}CH_2{-}CH_2{-}COOR} + 4H_2 \longrightarrow \underset{IV}{HO{-}(CH_2)_4{-}OH} + H_2O + ROH \qquad (3)$$

Der im Verlauf dieser Hydrierung freigesetzte Alkohol ROH kann destillativ abgetrennt und wieder zum Lösen des MSA oder genauer zur Erzeugung des Maleinsäurehalbesters II, in die Umsetzung zurückgeführt werden. Die Hydrierung von MSA in Gegenwart von Alkoholen verläuft somit gemäß Gesamtgleichung (4):

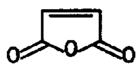 $+ 5 H_2 \longrightarrow HO-(CH_2)_4-OH + H_2O$ (4)

Als Nebenprodukt dieser Umsetzung kann sich Tetrahydrofuran (THF) entweder durch die nachträgliche Cyclisierung von BD bilden oder durch die direkte Hydrierung des intermediär in der Reaktionsmischung auftretenden γ-Butyrolactons (GBL).

MSA kann im erfindungsgemäßen Verfahren in fester, flüssiger oder gasförmiger Form verwendet werden. Zur Hydrierung wird es im jeweiligen Alkohol ROH gelöst und zwar zweckmäßigerweise bei Temperaturen von 20°C (Raumtemperatur) bis 100°C, vorzugsweise bei Temperaturen von 30 bis 70°C. Besonders vorteilhaft läßt sich gasförmiges MSA, wie es in der Regel im technischen Maßstab bei der katalytischen Oxidation von Butan, Buten oder aromatischen Kohlenwasserstoffen anfällt, einsetzen. Dabei kann das gasförmige MSA beispielsweise nach dem Verfahren der EP-A 149 144 in den betreffenden Alkoholen absorbiert werden. Das im technischen MSA enthaltene Wasser kann bei dieser Vorgehensweise besonders leicht abgetrennt werden, beispielsweise durch eine einfache, azeotrope Destillation. Es ist aber auch möglich, das so erhaltene Absorbat ohne Abtrennung des Wassers zu hydrieren.

Selbstverständlich können anstelle von MSA-Lösungen in Alkoholen auch Halb- und/oder Diester der Maleinsäure und Fumarsäure zur Hydrierung eingesetzt werden. Solche Gemische können als weitere Bestandteile GBL, Bernsteinsäure und/oder deren Halb- oder Diester enthalten. Die Verwendung derartiger Einsatzstoffe ist der Verwendung von MSA-Alkohol-Lösungen im erfindungsgemäßen Verfahren äquivalent.

Die erfindungsgemäße Hydrierung kann in Gegenwart der unterschiedlichsten Alkohole durchgeführt werden. Es können sowohl einwertige als auch mehrwertige, primäre, sekundäre oder tertiäre, aliphatische oder cycloaliphatische Alkohole verwendet werden. Bevorzugt ist jedoch die Verwendung von einwertigen, aliphatischen Alkoholen mit 1 bis 6 Kohlenstoffatomen, wie Methanol, Ethanol, Propanol, Isopropanol, n-Butanol, Isobutanol oder Hexanolen. Besonders vorteilhaft ist der Einsatz von Butanolen, da sich in ihrer Gegenwart die Abtrennung des im Zuge der Hydrierung entstandenen Wassers aus dem Hydrieraustrag durch Azeotropdestillation besonders einfach und effektiv gestalten läßt.

Das MSA wird in den betreffenden Alkoholen zweckmäßigerweise in einem Molverhältnis von 1:0,1 bis 1:30, vorzugsweise von 1:0,5 bis 1:20 und besonders bevorzugt in einem Molverhältnis von 1:1 bis 1:10 gelöst.

Erfindungsgemäß werden die MSA-Lösungen im betreffenden Alkohol zunächst in einer ersten Stufe bei Temperaturen von 100 bis 200°C, vorzugsweise bei Temperaturen von 150 bis 200°C und bei einem Druck von 30 bis 200 bar, bevorzugt bei einem Druck von 70 bis 150 bar, hydriert. Die Katalysatorbelastung kann dabei im allgemeinen 0,1 bis 10, insbesondere 1 bis 7 kg MSA/1 Katalysator und Stunde betragen.

Der Hydrieraustrag der ersten Stufe besteht in der Regel aus einem Gemisch der betreffenden Bernsteinsäuremonosäureester und Bernsteinsäurediester, dem jeweiligen Alkohol und Wasser. Der Bernsteinsäurediester entsteht zusammen mit dem Wasser bei den angewandten Hydrierbedingungen in Abhängigkeit von der Katalysatorbelastung. Im allgemeinen nimmt mit steigender Katalysatorbelastung die Menge des gebildeten Bernsteinsäurediesters im Hydrieraustrag ab. Außer den genannten Bernsteinsäureestern kann der Hydrieraustrag der ersten Hydrierstufe noch geringe Mengen - üblicherweise bis zu 10 Mol.-% bezogen auf das eingesetzte MSA - der ungesättigten Monoester und Diester der Maleinsäure und Fumarsäure enthalten. GBL, THF oder BD werden in der Regel in der ersten Hydrierstufe nicht oder nur in geringen Mengen gebildet.

Der Austrag der ersten Hydrierstufe wird erfindungsgemäß ohne Aufarbeitung der zweiten Hydrierstufe zugeführt.

In der zweiten Hydrierstufe wird der Hydrieraustrag der ersten Stufe erfindungsgemäß bei Temperaturen von 220 bis 300°C, vorzugsweise bei Temperaturen von 230 bis 260°C und bei einem Druck von 200 bis 350 bar, bevorzugt von 220 bis 300 bar, hydriert. Die Katalysatorbelastung kann 0,05 bis 0,9, insbesondere 0,2 bis 0,7 kg MSA/l Katalysator und Stunde betragen.

Der Hydrieraustrag der zweiten Stufe besteht im wesentlichen aus einem Gemisch aus BD, THF, Wasser und dem jeweiligen Alkohol. Weiterhin kann der Hydrieraustrag noch nicht umgesetztes GBL oder Bernsteinsäureester enthalten.

Die Wertprodukte können aus dem Hydrieraustrag nach den üblichen Aufarbeitungsmethoden, zweckmäßigerweise jedoch mit Hilfe destillativer Verfahren, isoliert werden. Die nicht vollständig hydrierten Zwischenprodukte der MSA-Hydrierung, wie Bernsteinsäureester, GBL und auch THF, können isoliert und den üblichen produktspezifischen Verwendungszwecken zugeführt werden oder aber vorteilhaft in die Hydrierung zurückgeleitet werden. Der zur Hydrierung von MSA zu BD benötigte Wasserstoff kann in stöchiometrischer Menge zudosiert werden, zweckmäßigerweise werden jedoch in beiden Hydrierstufen überschüssige Mengen an Wasserstoff eingesetzt. Dabei ist der Umfang des Wasserstoffüberschusses in der Regel nicht kritisch, da der unverbrauchte Wasserstoff im Kreis gefahren und wieder in die Hydrierung zurückgeführt wird. Falls gewünscht, kann ein Teil des Wasserstoffs als Abgas abgefackelt werden.

Die Hydrierungen der ersten und zweiten Hydrierstufe können jeweils in einem oder mehreren Reaktoren durchgeführt werden. Mit dem Begriff "Hydrierstufe" wird somit die Hydrierung unter den Bedingungen eines bestimmten Temperatur- und Druckbereiches verstanden. Der Hydriervorgang einer Hydrierstufe kann sich somit über mehrere Reaktoren ausdehnen, d.h. eine Hydrierstufe kann apparativ aus mehreren Reaktoren bestehen.

Das erfindungsgemäße Verfahren kann diskontinuierlich betrieben werden, vorzugsweise wird jedoch kontinuierlich verfahren. Zur kontinuierlichen Verfahrensweise können vorteilhaft Rohr- oder Rohrbündelreaktoren eingesetzt werden, wobei nach der Sumpf- oder Rieselfahrweise gearbeitet werden kann. Die Reaktoren können mit Hilfe der üblichen Vorrichtungen zur Temperaturregulierung, also zur Beheizung und Kühlung von innen oder außen, versehen sein. Eine weitere Möglichkeit zur Temperaturregulierung besteht in der Rückführung von unverbrauchtem Wasserstoff oder eines Teils des Hydrieraustrages der zweiten Stufe in die Hydrierung.

Die Katalysatoren können in suspendierter Form angewandt werden, bevorzugt wird jedoch die Festbettanordnung der Katalysatoren.

Prinzipiell können im erfindungsgemäßen Verfahren alle Katalysatoren verwendet werden, die zur Hydrierung von MSA, MS, FS sowie den Monoestern und Diestern dieser Säuren zu BD geeignet sind. Solche Katalysatoren sind beispielsweise in DE-A 25 19 817, EP-B 147 219, DE-A 28 45 905, US-A 4 810 807 und DE-A 19 01 870 beschrieben.

Besonders geeignet sind für das erfindungsgemäße Verfahren solche Katalysatoren, die Kobalt und mindestens eines der Elemente Mangan, Kupfer und/oder Phosphor enthalten. Bevorzugt werden Katalysatoren benutzt, welche neben Kobalt noch mindestens zwei der Elemente Mangan, Kupfer, Phosphor und/oder Molybdän enthalten. Durch besonders vorteilhafte Eigenschaften zeichnen sich im erfindungsgemäßen Verfahren solche Katalysatoren aus, die neben Kobalt mindestens drei der Elemente Mangan, Kupfer, Phosphor, Molybdän und/oder Natrium enthalten. Solche Katalysatoren sind bekannt und in DE-A 23 21 101 sowie in der DE-A 39 04 083 beschrieben. Vorteilhaft können im erfindungsgemäßen Verfahren beispielsweise solche Katalysatoren angewandt werden, deren katalytisch aktive Masse zu mindestens 40 Gew.-% aus Kobalt (ber. als Co) besteht und als weitere katalytische aktive Bestandteile bis zu 10 Gew.-%, vorzugsweise 3 bis 7 Gew.-% Mangan (ber. als Mn), bis zu 20 Gew.-% vorzugsweise 0,1 bis 5 Gew.-% Phosphorsäure ($H_3PO_4$) und bis zu 1 Gew.-%, vorzugsweise 0,01 bis 0,5 Gew.-% Natrium (ber. als Na) enthält. Besonders bevorzugt werden solche der vorgenannten Katalysatoren verwendet, deren katalytisch aktive Masse als zusätzliche katalytisch aktive Bestandteile bis zu 30 Gew.-%, vorzugsweise 12 bis 18 Gew.-% Kupfer (ber. als Cu) und bis zu 5 Gew.-%, vorzugsweise 1 bis 4 Gew.-% Molybdän (ber. als Mo) enthält.

Die erfindungsgemäß verwendeten Katalysatoren können im erfindungsgemäßen Verfahren sowohl in Form von Trägerkatalystoren oder vorzugsweise in kompakter Form, d.h. ohne Träger zur Anwendung gelangen. Die Art des Trägermaterials ist in der Regel nicht kritisch, es können herkömmliche Trägermaterialien wie Siliciumdioxid, Aluminiumoxide, Titandioxide, Aktivkohle, Silikate oder Zeolithe verwendet werden. Erforderlichenfalls können zur Herstellung der Katalystoren Bindemittel und Formhilfsmittel mitverwendet werden.

Die Katalysatoren werden vorzugsweise vor ihrem Einsatz im erfindungsgemäßen Verfahren mit Wasserstoff aktiviert. Dabei werden die nach der Kalzinierung im allgemeinen in Form ihrer Oxide vorliegenden katalytisch aktiven Katalysatorbestandteile größtenteils reduziert und zwar in der Regel zu den entsprechenden Metallen. Weitere Einzelheiten zur Herstellung dieser Katalysatoren können DE-A 23 21 101 sowie der DE-A 39 04 083 entnommen werden.

Das erfindungsgemäße, zweistufige Hydrierverfahren zur Herstellung von BD aus MSA ermöglicht die Ableitung von bis zu 50 % der dabei anfallenden Hydrierwärme bei tieferen Temperaturen als im Einstufenverfahren. Dadurch wird die Gefahr der "hot-spot-Bildung", die insbesondere bei hohen Hydriertemperaturen und hohen Katalysatorbelastungen besteht, vermindert und die Selektivität und Standzeit des Katalysators, insbesondere bei der Ausübung des Verfahrens im industriellen Maßstab, erhöht.

Beispiel 1

Die Hydrierung wurde mit einer Hydrieranlage gemäß der beiliegenden Zeichnung ausgeführt.

In das Reaktorrohr A der in der Zeichnung schematisch dargestellten Reaktorkaskade, wurden 0,49 kg Katalysator eingebaut. Die Länge des Reaktorrohres betrug 2000 mm und sein Innendurchmesser 30 mm. Der verwendete Katalysator enthielt die folgenden katalytisch aktiven Bestandteile: 63,4 Gew.-% Kobalt, berechnet als CoO, 18,1 Gew.-% Kupfer, berechnet als CuO, 6,8 Gew.-% Mangan, berechnet als $Mn_3O_4$, 3,1 Gew.-% Molybdän, berechnet als $MoO_3$, 0,15 Gew.-% Natrium, berechnet als $Na_2O$ und 3,3 Gew.-% Phosphor, berechnet als $H_3PO_4$. Der Katalysator bestand aus 4 mm starken Strangpreßlingen. In das Reaktorrohr B der Kaskade,

mit einer Länge von 2000 mm und einem Innendurchmesser von 30 mm, wurden 1,49 kg und in das Reaktorrohr C, mit einer Länge von 2000 mm und einem Innendurchmesser von 16 mm, wurden 0,38 kg des oben beschriebenen Katalysators eingebaut.

Die Reduktion des Katalysators wurde in jedem Reaktorrohr separat durchgeführt. Hierzu wurden die Rohre mit einer Heizrate von 20°C/min unter Zuleitung von 300 l/h Stickstoff auf 290°C aufgeheizt. Anschließend wurde innerhalb von 6 Stunden der Stickstoff langsam gegen Wasserstoff ausgetauscht. Danach wurde die Temperatur auf 300 bis 310°C erhöht und 48 Stunden lang reiner Wasserstoff bei dieser Temperatur über den Katalysator geleitet und zwar in einer Menge von 300 l/h.

Die erste Stufe der Hydrierung wurde in Reaktor A durchgeführt, in den Reaktoren B und C erfolgte die zweite Stufe der Hydrierung. Zur Hydrierung wurde nach der Reduktion des Katalysators ein Gemisch aus MSA und n-Butanol in einem Molverhältnis von 1:2,5 und in einer Menge von 1,23 kg/h mit der Zulaufpumpe (2) aus dem Behälter (1) zusammen mit Wasserstoff in den Hydrierkreislauf des Reaktors A geleitet. Der Hydrieraustrag der ersten Hydrierstufe wurde aus dem Reaktor A über den Gas-Flüssigkeit-Abscheider (7) mit der Pumpe (4) standgeregelt in den Hydrierkreislauf des Reaktors B geleitet. Der Hydrieraustrag des Reaktors B wurde über den Gas-Flüssigkeit-Abscheider (8) standgeregelt mit Hilfe der Pumpe (6) in den Reaktor C transportiert. Das Hydrierprodukt wurde aus dem Reaktor C über den Gas-Flüssigkeit-Abscheider (9) ausgetragen. Die Pumpen (3) und (5) dienten zur Im-Kreis-Führung der Reaktionsmischung in den Hydrierkreisläufen der Reaktoren A (Hydrierstufe 1) bzw. B (1. Teil der Hydrierstufe 2). Der über die drei Gas-Flüssigkeit-Abscheider (7), (8) und (9) von der flüssigen Reaktionsmischung abgetrennte Wasserstoff konnte über die Leitungen (10), (11) bzw. (12) und die Ventile (13), (14) bzw. (15) in die Reaktoren A, B bzw. C zurückgeleitet oder als Abgas abgeführt werden. Im vorliegenden Beispiel wurde der Wasserstoff in jeden Reaktor separat eindosiert und der überschüssige Wasserstoff über die betreffenden Abscheider ausgekreist und als Abgas abgefackelt. Die Reaktorrohre wurden elektrisch mittels Blockheizungen beheizt.

Es wurden die folgenden Reaktionsbedingungen eingestellt:

Tabelle 1: Reaktionsbedingungen

| | Reaktor A | Reaktor B | Reaktor C |
|---|---|---|---|
| Druck (bar) | 100 | 230 | 260 |
| Temperatur (°C) | 190 | 260 | 260 |
| Abgasmenge (l/h) | 110 | 400 | 300 |
| Kreislaufmenge (l/h) | 10 | 10 | - |
| Wasserstoffmenge (l/h) | 220 | 780 | 360 |

In Reaktor A wurde der Katalysator mit 1,74 kg MSA/l Katalysator und Stunde, bezogen auf die zugeführte MSA-Menge, belastet. Für die zweite Hydrierstufe - also die Reaktoren B und C - ergab sich unter den angewandten Bedingungen, bezogen auf das gesamte Katalysatorvolumen der Reaktoren B und C, eine Katalysatorbelastung von 0,45 kg MSA/l Katalysator und Stunde. Die Katalysatorbelastung des gesamten Katalysatorvolumens beider Hydrierstufen betrug 0,36 kg MSA/l Katalysator und Stunde.

Gemäß dem Ergebnis der gaschromatographischen Untersuchung der Hydrierausträge hatten diese die folgende Zusammensetzung:

Tabelle 2: Zusammensetzung der Hydrierausträge nach der ersten und zweiten Hydrierstufe

| | Nach Reaktor A | Nach Reaktor C |
|---|---|---|
| Bernsteinsäuredibutylester | 15,4 Mol.-% | 6,9 Mol.-% |
| Bernsteinsäuremonobutylester | 84,6 Mol.-% | 0,3 Mol.-% |
| Butandiol (BD) | 0,0 Mol.-% | 68,9 Mol.-% |
| γ-Butyrolacton (GBL) | 0,0 Mol.-% | 10,6 Mol.-% |
| THF | 0,0 Mol.-% | 8,4 Mol.-% |

Der Umsatz zu BD, GBL und THF betrug somit 87,9 Mol.-%. Unter Berücksichtigung der Rückführbarkeit der Bernsteinsäureester in die Hydrierung ergibt sich hieraus nach der folgenden Rechenformel

$$\text{Gesamtselektivität} = \frac{\text{Mole BD} + \text{Mole GBL} + \text{Mole THF}}{\text{Mole MSA} - \text{Mole Bernsteinsäureester}} \bullet 100$$

eine Gesamtselektivität für BD, GBL und THF von 94,4 %.

Vergleichsbeispiel

Die Reaktorrohre A, B und C der Apparatur gemäß Beispiel 1 wurden wie folgt mit dem Katalysator gemäß Beispiel 1 befüllt:
Reaktorrohre A und B mit jeweils 1,38 kg;
Reaktorrohr C mit 0,36 kg.

Anschließend wurde der Katalysator, wie in Beispiel 1 beschrieben, reduziert. Nach der Reduktion wurde ein Gemisch von MSA in n-Butanol im Molverhältnis 1:2,5 und in einer Menge von 1 kg/h in die Reaktorkaskade geleitet. Alle drei Reaktoren wurden im Temperaturbereich von 240 bis 260°C und im Druckbereich von 200 bis 260 bar gehalten. Im übrigen wurde die Hydrierapparatur, wie in Beispiel 1 beschrieben, betrieben.

Es wurden die folgenden Reaktionsbedingungen eingestellt:

Tabelle 3: Reaktionsbedingungen

| | Reaktor A | Reaktor B | Reaktor C |
|---|---|---|---|
| Druck (bar) | 200 | 230 | 260 |
| Temperatur (°C) | 240 | 240 | 260 |
| Abgasmenge (l/h) | 300 | 300 | 300 |
| Kreislaufmenge (l/h) | 10 | 10 | - |
| Wasserstoffmenge (l/h) | 410 | 610 | 340 |

Der Katalysator im Reaktor A wurde mit 0,5 kg MSA/l Katalysator und Stunde, bezogen auf die zugeführte MSA-Menge, belastet. Die Katalysatorbelastung des gesamten Katalysatorvolumens der Hydrierapparatur betrug 0,22 kg MSA/l Katalysator und Stunde.

Die Hydrierausträge der Reaktoren A und C hatten nach dem Ergebnis ihrer gaschromatographischen Analyse die folgende Zusammensetzung:

Tabelle 4: Zusammensetzungen der Hydrierausträge der Reaktoren A und C

| | Nach Reaktor A | Nach Reaktor C |
|---|---|---|
| Bernsteinsäuredibutylester | 20,5 Mol.-% | 3,0 Mol.-% |
| Bernsteinsäuremonobutylester | 34,6 Mol.-% | 0,1 Mol.-% |
| Butandiol (BD) | 10,0 Mol.-% | 61,6 Mol.-% |
| γ-Butyrolacton (GBL) | 22,3 Mol.-% | 4,5 Mol.-% |
| THF | 12,7 Mol.-% | 21,0 Mol.-% |

Unter Berücksichtigung der Rückführbarkeit der Bernsteinsäureester in die Hydrierung ergibt sich hieraus eine Gesamtselektivität für BD, GBL und THF von 89,8 %.

Daraus folgt, daß die Hydrierung von MSA in zwei Hydrierstufen gemäß Beispiel 1 trotz einer höheren Katalysatorbelastung eine höhere Gesamtselektivität ergibt als die Hydrierung von MSA in nur einer Hydrierstufe.

## Patentansprüche

1. Verfahren zur Herstellung von 1,4-Butandiol durch die katalytische Hydrierung von Maleinsäureanhydrid in Gegenwart von Alkoholen, bei erhöhter Temperatur und bei erhöhtem Druck und mit Hilfe eines kobalthaltigen Katalysators, dadurch gekennzeichnet, daß man die Reaktionsmischung in einer ersten

Hydrierstufe bei Temperaturen von 100 bis 200°C und einem Druck von 30 bis 200 bar teilhydriert und anschließend den Hydrieraustrag dieser ersten Stufe ohne weitere Aufarbeitung bei höheren Temperaturen und bei einem höheren Druck als in der ersten Hydrierstufe in einer zweiten Hydrierstufe bei Temperaturen von 200 bis 300°C und einem Druck von 200 bis 350 bar nachhydriert.

2. Verfahren nach Anspruch 1, dadurch gekennzeichnet, daß man einen Katalysator verwendet, der Kobalt und mindestens eines der Elemente Mangan, Kupfer und/oder Phosphor enthält.

3. Verfahren nach Anspruch 1, dadurch gekennzeichnet, daß man einen Katalysator verwendet, der Kobalt und mindestens zwei der Elemente Mangan, Kupfer, Phosphor und/oder Molybdän enthält.

4. Verfahren nach Anspruch 1, dadurch gekennzeichnet, daß man einen Katalysator verwendet, der Kobalt und mindestens drei der Elemente Mangan, Kupfer, Phosphor, Molybdän und/oder Natrium enthält.

5. Verfahren nach Anspruch 1, dadurch gekennzeichnet, daß die aktive Masse des Katalysators zu mindestens 40 Gew.-% aus Kobalt (ber. als Co) besteht.

6. Verfahren nach Anspruch 1, dadurch gekennzeichnet, daß man einen Katalysator verwendet, dessen aktive Masse zu mindestens 40 Gew.-% aus Kobalt (ber. als Co) besteht und als weitere katalytisch aktive Bestandteile bis zu 10 Gew.-% Mangan (ber. als Mn), bis zu 20 Gew.-% Phosphorsäure und bis zu 1 Gew.-% Natrium (ber. als Na) enthält.

7. Verfahren nach Anspruch 1, dadurch gekennzeichnet, daß man einen Katalysator verwendet, dessen aktive Masse zu mindestens 40 Gew.-% aus Kobalt (ber. als Co) besteht und als weitere katalytische aktive Bestandteile bis zu 10 Gew.-% Mangan (ber. als Mn), bis zu 30 Gew.-% Kupfer (ber. als Cu), bis zu 5 Gew.-% Molybdän (ber. als Mo), bis zu 20 Gew.-% Phosphorsäure und bis zu 1 Gew.-% Natrium (ber. als Na) enthält.

8. Verfahren nach Anspruch 1, dadurch gekennzeichnet, daß man als Alkohol n-Butanol verwendet.

## Claims

1. A process for the preparation of 1,4-butanediol by catalytically hydrogenating maleic anhydride in the presence of an alcohol, at elevated temperature and elevated pressure and with the aid of a cobalt-containing catalyst, which comprises partially hydrogenating the reaction mixture in a first hydrogenation step at from 100 to 200°C and at from 30 to 200 bar and subsequently post-hydrogenating the product of this first hydrogenation step, without further work-up at higher temperatures and pressure than in the first hydrogenation step, in a second hydrogenation step at from 200 to 300°C and at from 200 to 350 bar.

2. A process as claimed in claim 1, wherein the catalyst used contains cobalt and at least one of the elements manganese, copper and/or phosphorus.

3. A process as claimed in claim 1, wherein the catalyst used contains cobalt and at least two of the elements manganese, copper, phosphorus and/or molybdenum.

4. A process as claimed in claim 1, wherein the catalyst used contains cobalt and at least three of the elements manganese, copper, phosphorus, molybdenum and/or sodium.

5. A process as claimed in claim 1, wherein the active material of the catalyst comprises at least 40% by weight of cobalt (calculated as Co).

6. A process as claimed in claim 1, wherein a catalyst is used whose active material comprises at least 40% by weight of cobalt (calculated as Co) and which contains, as further catalytically active constituents, up to 10% by weight of manganese (calculated as Mn), up to 20% by weight of phosphoric acid and up to 1% by weight of sodium (calculated as Na).

7. A process as claimed in claim 1, wherein a catalyst is used whose active material comprises at least 40% by weight of cobalt (calculated as Co) and which contains, as further catalytically active constituents, up to 10% by weight of manganese (calculated as Mn), up to 30% by weight of copper (calculated as Cu),

up to 5% by weight of molybdenum (calculated as Mo), up to 20% by weight of phosphoric acid and up to 1% by weight of sodium (calculated as Na).

8. A process as claimed in claim 1, wherein the alcohol used is n-butanol.

## Revendications

1. Procédé de préparation de butanediol-1,4 par l'hydrogénation catalytique d'anhydride maléique en présence d'alcools, à température élevée, sous pression élevée et à l'aide d'un catalyseur contenant du cobalt, caractérisé en ce qu'on soumet le mélange réactionnel à une hydrogénation partielle, dans une première étape d'hydrogénation à une température de 100 à 200°C et sous une pression de 30 à 200 bar, puis on soumet le produit d'hydrogénation de cette première étape, sans autre traitement ultérieur, a une post-hydrogénation à une température et sous une pression plus élevées que dans la première étape d'hydrogénation, dans une seconde étape d'hydrogénation à une température de 200 à 300°C et sous une pression de 200 à 350 bar.

2. Procédé selon la revendication 1, caractérisé en ce qu'on utilise un catalyseur qui contient du cobalt et l'un au moins des éléments manganése, cuivre et/ou phosphore.

3. Procédé selon la revendication 1, caractérisé en ce qu'on utilise un catalyseur qui contient du cobalt et au moins deux des éléments manganèse, cuivre, phosphore et/ou molybdène.

4. Procédé selon la revendication 1, caractérisé en ce qu'on utilise un catalyseur qui contient du cobalt et au moins trois des éléments manganèse, cuivre, phosphore, molybdène et/ou sodium.

5. Procédé selon la revendication 1, caractérisé en ce que la masse active du catalyseur se compose, pour au moins 40% en poids, de cobalt (calculés en tant que Co).

6. Procédé selon la revendication 1, caractérisé en ce qu'on utilise un catalyseur dont la masse active se compose, pour au moins 40% en poids, de cobalt (calculés en tant que Co) et contient, comme autres constituants à activité catalytique, jusqu'à 10% en poids de manganèse (calculés en tant que Mn), jusqu'à 20% en poids d'acide phosphorique et jusqu'à 1% en poids de sodium (calculé en tant que Na).

7. Procédé selon la revendication 1, caractérisé en ce qu'on utilise un catalyseur dont la masse active se compose, pour au moins 40% en poids, de cobalt (calculés en tant que Co) et contient, comme autres constituants à activité catalytique, jusqu'à 10% en poids de manganèse (calculés en tant que Mn), jusqu'a 30% en poids de cuivre (calculés en tant que Cu), jusqu'à 5% en poids de molybdéne (calculés en tant que Mo), jusqu'à 20% en poids d'acide phosphorique et jusqu'à 1% en poids de sodium (calculé en tant que Na).

8. Procédé selon la revendication 1, caractérisé en ce qu'on utilise, comme alcool, du n-butanol.

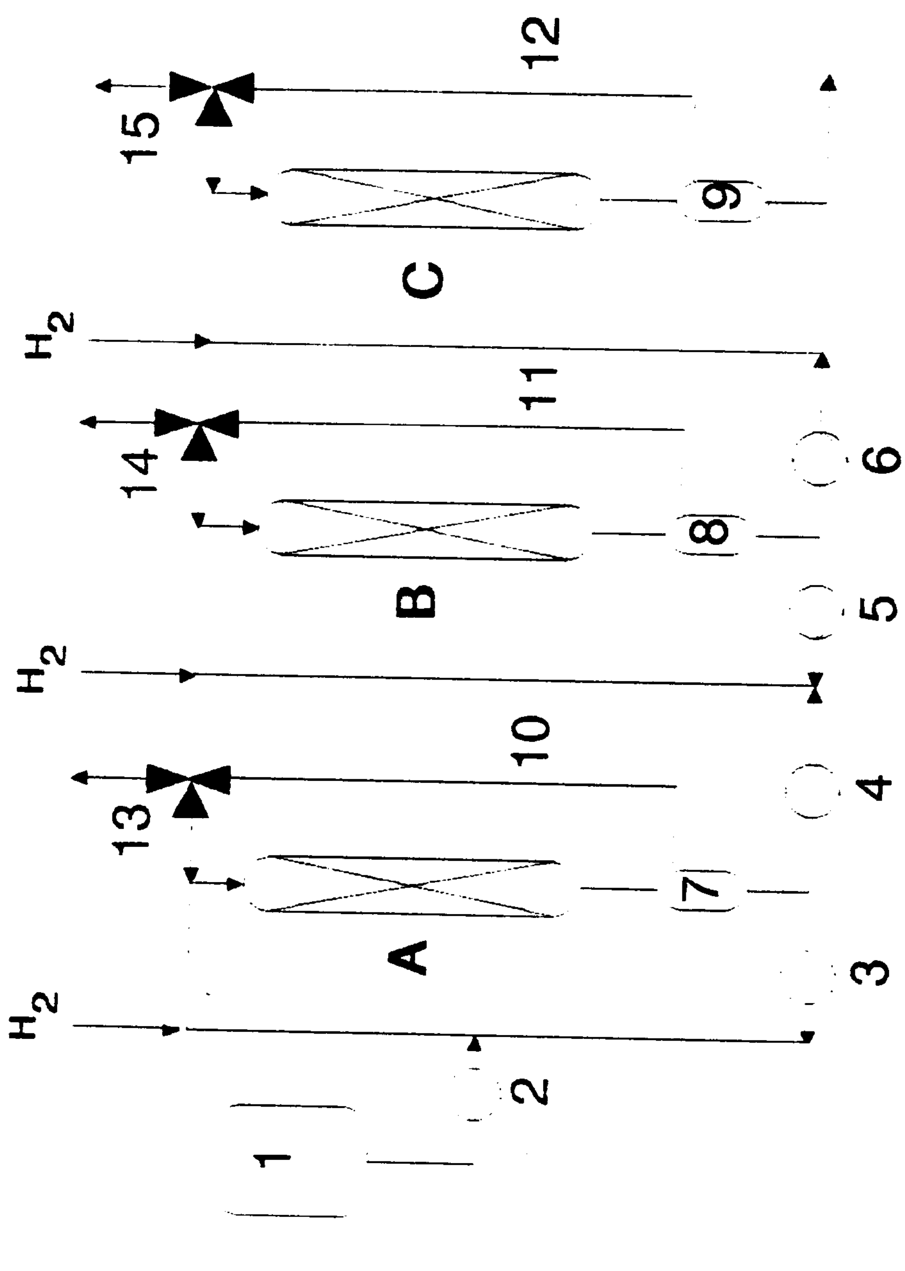
A
B
C
$H_2$
$H_2$
$H_2$
1
2
3
4
5
6
7
8
9
10
11
12
13
14
15